(19) Europäisches Patentamt
European Patent Office
Office européen des brevets

(11) Numéro de publication: **0 469 968 B1**

(12) # FASCICULE DE BREVET EUROPEEN

(45) Date de publication de fascicule du brevet: **21.06.95**

(51) Int. Cl.$^{6}$: **A61K 31/70**

(21) Numéro de dépôt: **91402080.5**

(22) Date de dépôt: **25.07.91**

(54) **Composition contenant une adénosine substituée en N6 pour le traitement de l'hyperlipémie ou de l'hypertriglycéridémie.**

(30) Priorité: **03.08.90 FR 9009957**

(43) Date de publication de la demande:
**05.02.92 Bulletin 92/06**

(45) Mention de la délivrance du brevet:
**21.06.95 Bulletin 95/25**

(84) Etats contractants désignés:
**AT BE CH DE DK ES FR GB GR IT LI LU NL SE**

(56) Documents cités:

**EP-A- 0 186 574** **EP-A- 0 322 242**
**DE-A- 2 406 587** **FR-A- 2 111 862**
**FR-A- 2 239 252** **GB-A- 2 077 725**

**RESEARCH COMMUNICATIONS IN CHEMICAL PATHOLOGY AND PHARMACOLOGY, vol. 68, no. 3,juin 1990, pages 299-305; J.F. BONIFACJ et al.: "Action of N6(amido-3-propyl)adenosine hydrochloride (Agr 529) on plasma corticosterone levels in rats"**

(73) Titulaire: **CENTRE D'ETUDES EXPERIMENTALES ET CLINIOUES DE PHYSIO-BIOLOGIE, DE PHARMACOLOGIE ET D'EUTONOLOGIE (CEPBEPE)**
**78, rue de la Convention**
**F-75015 Paris (FR)**

(72) Inventeur: **Laborit,Geneviève**
**93 rue de la Santé**
**F-75013 Paris (FR)**
Inventeur: **Wermuth,Camille**
**3 rue de la Côte d'Azur**
**F-67100 Strasbourg (FR)**

(74) Mandataire: **Bloch, Gérard et al**
**2, square de l'Avenue du Bois**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 469 968 B1

## Description

La présente invention concerne l'application d'une substance contenant, comme principe actif, une adénosine substituée en $N^6$, pour l'obtention d'un médicament destiné au traitement de l'hyperlipémie et de l'hypertriglycéridémie.

On a décrit dans le brevet FR-A-8419047 l'action particulièrement forte, hypotensive ou anti-hypertensive, anti-inflamatoire, analgésique, d'une adénosine substituée, de formule I

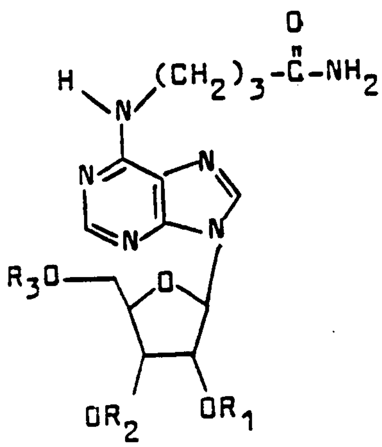

(I)

dans laquelle $R_1$, $R_2$ et $R_3$ représentent chacun l'hydrogène (Agr 529) ou

$$CH_3 - CH_2 - \overset{O}{\overset{\|}{C}} - \quad \text{(Agr 540)}.$$

Or on a trouvé que l'administration de l'Agr 529 ($N_6$-carboxamido-3-propyl)adénosine et de sa prodrogue Agr 540 [(carboxamido-3-propylamino)-6-(tripropionyl 2′,3′,5′,$\beta$, D ribosyl)-9 purine] provoque chez les animaux normaux une hypolipémie (baisse des acides gras, des phospholépides des triglycérides et du cholestérol) et entraîne une normalisation de l'hypertriglycéridémie provoquée.

L'invention propose donc l'application d'Agr 529 et d'Agr 540, comme ingrédient actif pour l'obtention d'un médicament destiné au traitement de l'hyperlipémie et de l'hypertriglycédémie.

L'expérimentation a donc porté, d'une part, sur des animaux sur lesquels une hypertriglycéridémie a été provoquée, d'autre part, sur des animaux normaux.

<u>TEST D'HYPERTRIGLYCERIDEMIE PROVOQUEE.</u>

L'expérimentation porte sur deux espèces d'animaux :

- lapins Fauve de Bourgogne d'un poids moyen de 2 kg (EGAV).
- rats blancs mâles Sprague-Dawley, Iffa Credo, d'un poids moyen de 260-270 g.

L'hypertriglycéridémie est obtenue par injection intraveineuse (iv) de Triton WR1339 (tyloxapol), à la dose de 600 $mg.kg^{-1}$, provenant des Etablissements Sigma Chemical Company, N. T 8761 (veine marginale de l'oreille sur le lapin, veine jugulaire ou veint du pénis sur le rat, sous anesthésie légère à l'éther, le pentobarbital qui se fixe sur les récepteurs A1 pouvant interagir avec les molécules utilisées (Lhose et al., 1985). Tous les animaux utilisés sont à jeûn depuis quinze heures. Le sang est collecté sur héparine, centrifugé vingt minutes (3000 RPM, à 4 degrés C). Dans le sérum recueilli, les TG (triglycérides) sont dosés suivant la méthode GPO-PAP Kit Boeringher Peridochrom(R), référence : 701-882.

Chez les animaux normaux, les acides gras, les triglycérides, le cholestérol sont dosés par réactions enzymatiques automatisées.

1. Expérimentation sur le lapin.

Les animaux sont divisés en deux lots :

- un lot témoin (6 animaux) recevant en iv du soluté salé isotonique sous un volume de 0,5 $ml.kg^{-1}$, deux heures avant l'injection de Triton et la même dose de soluté salé que précédemment.
- un lot traité (7 animaux) recevant en iv 2 $mg.kg^{-1}$ d'Agr 529 sous un volume de 0,5 $ml.kg^{-1}$, deux heures avant l'injection du Triton et d'une nouvelle dose d'Agr 529 (2 $mg.kg^{-1}$).

Un prélèvement de sang artériel, deux heures après l'injection du Triton, permet le dosage des TG.

2. Expérimentation par voie intrapéritonéale sur le rat.

Les animaux sont divisés en deux lots:

- un lot témoin (4 animaux) recevant du soluté salé isotonique sous un volume de 0,2 ml/100 g rat en injection intrapéritonéale (ip), vingt minutes avant l'injection du Triton (iv).
- un lot traité (5 animaux) recevant 2 $mg.kg^{-1}$ d'Agr 529 en ip, dans les mêmes conditions que les témoins.

Deux heures après, les animaux sont sacrifiés par décapitation; le sang est recueilli suivant la méthode précédemment décrite, pour le dosage des TG.

3. Expérimentation per os sur le rat.

Les animaux sont divisés en six lots :

- un lot témoin-témoin recevant, per os, du soluté salé isotonique sous un volume de 0,2 ml/100 g rat, trente minutes avant l'injection IV de soluté salé isotonique.
- un lot témoin-Triton recevant per os du soluté salé isotonique, trente minutes avant l'injection IV du Triton.
- un lot Agr 529 (3 $mg.kg^{-1}$ sous un volume de 0,2 ml/100 g rat), trente minutes avant l'injection IV du Triton.
- un lot recevant dans les mêmes conditions 30 $mg.kg^{-1}$ d'Agr 529, per os.
- deux lots recevant dans les mêmes conditions que précédemment 3 et 30 $mg.kg^{-1}$ d'Agr 540 per os.

EXPERIMENTATION CHEZ LES ANIMAUX NORMAUX.

1. Administration intraveineuse :

Les animaux sont répartis en six lots de cinq animaux :

- un lot témoin recevant le placebo (soluté salé isotonique à 0,9 %).
- cinq lots traités recevant respectivement 0,03, 0,1, 0,3, 1 et 3 $mg.kg^{-1}$ d'Agr 529 par voie intraveineuse. Les animaux sont sacrifiés deux heures après l'injection du produit.

2. Administration intrapéritonéale :

Les animaux sont répartis en cinq lots de cinq animaux :

- un lot témoin.
- quatre lots traités recevant 0,1, 0,3, 1 et 3 $mg.kg^{-1}$.

Les animaux sont sacrifiés deux heures après l'administration de l'Agr 529.

3. Significativité.

L'étude statistique est réalisée à l'aide du test du t de Student avec : * $P < 0,05$; ** $P < 0,01$; *** $P < 0,001$ ou par le test de Dunnett avec * $P < 0,05$ et ** $P < 0,01$.

RESULTATS

HYPERTRIGLYCERIDEMIE PROVOQUEE.

1. Chez le lapin en intraveineux.

Chez les animaux témoins, le Triton provoque une augmentation significative (***) des TG de 196,5 %, alors que chez les animaux ayant reçu préalablement de l'Agr 529 (2 $mg.kg^{-1}$, iv), l'augmentation est de 45 % (*). Comparés aux témoins Triton et au temps 4, les animaux ayant reçu l'Agr 529 ont un taux de TG de 150 % inférieur à celui des témoins (voir tableau I).

2. Chez le rat.

a) L'Agr 529 en injection intrapéritonéale.

L'injection d'Agr 529 en ip provoque une diminution de 54 % du taux circulant des TG. Cette action de l'Agr 529 est significative (**) (voir tableau II).

b) L'Agr 529 en administration per os.

Per os, l'Agr 529 et l'Agr 540 s'opposent significativement à l'augmentation des TG circulants. L'action la plus importante est obtenue avec 30 $mg.kg^{-1}$ (51 % d'inhibition; voir tableau III). On notera que l'action de l'Agr 529 est sensiblement la même que celle de l'Agr 540, malgré le fait que ce dernier soit mieux absorbé per os (Rapport communiqué sur l'Agr 529). Sans doute, cela est-il dû au fait que de petites doses (nanomolaires) soient suffisantes à l'action sur les A1 récepteurs. On peut envisager d'utiliser des doses plus faibles des deux composés dans le modèle développé, puisqu'on ne fait pas varier significativement l'action obtenue en augmentant la dose de 3 à 30 $mg.kg^{-1}$, tout au moins en ce qui concerne l'Agr 529.

B. RATS NORMAUX.

a) L'Agr 529 en intraveineux.

Dans la gamme des doses utilisées, l'effet hypolipémiant ne se manifeste qu'à partie de 0,1 $mg.kg^{-1}$. A 3 $mg.kg^{-1}$ on note une diminution significative, deux heures après l'injection, des taux de triglycérides, de cholestérol total, d'acides gras, de phospholipides plasmatiques.

b) L'Agr 529 en intrapéritonéale.

On retrouve les mêmes actions que par voie iv mais l'effet hypolipémiant ne commence à être significatif qu'à partir de 0,3 $mg.kg^{-1}$ (figures I, II, III, IV).

c) Etude cinétique de l'Agr 529 à 3 $mg.kg^{-1}$ iv (figures V, VI, VII, VIII).

L'administration de 3 $mg.kg^{-1}$ d'Agr 529 entraîne une baisse significative des taux d'acides gras, de triglycérides, de cholestérol total, de phospholipides. L'effet commence à se manifester trente minutes après l'injection et s'accentue dans le temps. La baisse est maximale au bout de deux heures. Huit heures après l'administration, il y a une tendance de retour aux taux de base. Ce qui indique que l'action de l'Agr 529 est de longue durée et que celui-ci n'est pas détruit par l'adénosine déaminase.

En résumé, utilisant un modèle d'hypertriglycéridémie provoquée par l'injection intraveineuse de Triton WR 1339, on a constaté que l'Agr 529 [chlorhydrate de N(amido-3-propyl-adénosine] à 2 $mg.kg^{-1}$ par voie intraveineuse chez le lapin d'une part et par voie intrapéritonéale d'autre part normalisait le taux plasmatique des triglycérides circulants.

Par ailleurs l'Agr 529 et sa prodrogue, l'Agr 540 [(carboxamido-3-propylamino)-6-(tripropionyl) 2′, 3′, 5′,$\beta$, D ribosyl)-9-purine] administrés per os aux doses de 3 et 30 $mg.kg^{-1}$ chez le rat ramènent à la normale les concentrations plasmatiques de triglycérides.

L'administration intraveineuse d'Agr 529 à des rats normaux provoque en fonction des doses une diminution des taux plasmatiques des acides gras, des phospholipides, des triglycérides, du cholestérol total. La diminution débute à la dose de 0,1 $mg.kg^{-1}$ et devient hautement significative à 3 $mg.kg^{-1}$, dose à

laquelle on note par ailleurs une hyperglycémie. Dans les mêmes conditions, l'administration intrapéritonéale d'Agr 529 entraîne également en fonction des doses une hypolipémie. Cependant à 0,3 $mg.kg^{-1}$, on ne retrouve plus d'hyperglycémie. Aucune action n'est à signaler sur les taux de cholestérol et de triglycérides des lipoprotéines à haute densité (HDL).

Une étude cinétique montre que l'action hypolipémiante de l'Agr 529 à 3 $mg.kg^{-1}$ en injection intraveineuse se manifeste trente minutes après l'injection avec un effet maximum à deux heures. L'effet persiste pendant les huit heures qui suivent l'injection.

Une étude in vitro sur fraction microsomique de foie révèle que l'Agr 529 n'a aucune action sur l'activité de la HMG CoA réductase ( $\beta$-hydroxy-$\beta$-méthylglutaryl CoA réductase : enzyme clef de la synthèse du cholestérol). Il en est de même sur l'activité de l'ACAT (cholestérol-O-acyl transférase), enzyme de l'estérification du cholestérol.

En conclusion, on démontre que l'administration d'Agr 529 et d'Agr 540 provoque chez les animaux normaux une hypolipémie (baisse des acides gras, des phospholipides, des triglycérides et du cholestérol) et entraîne une normalisation de l'hypertriglyvéridémie provoquée.

Les doses actives pour observer les effets objets de la présente demande sont inférieures par plusieurs facteurs à celles qui produisent les effets objets du brevet FR 84 19 047. Ces derniers ne sont pas observés ou sont négligeables aux doses hypolipémiantes.

Tableaux I, II, III
Figures I à VIII

TABLEAU I

Action de l'Agr 529 à la dose de 2 mg.kg-1, administré par voie intraveineuse (iv) sur l'hypertriglycéridémie provoquée par l'injection par la même voie de 600 mg.kg-1 de Triton chez le lapin Fauve de Bourgogne (n: nombre d'animaux).

| Dosage<br>Animaux | Triglycérides mmole/litre temps= 0 | Produit injecté | Latence (heures) | Triglycérides mmole/litre temps= 2 | Produit injecté | Latence (heures) | Triglycérides mmole/litre temps= 4 |
|---|---|---|---|---|---|---|---|
| Témoins n=6 | 1,74±0,16 | Solvant NaCl 0,9% iv | 2 | 2,98±0,40 | Solvant NaCl 0,9%, iv + Triton 600 mg.kg-1 iv | 2 | 8,83±1,13 |
| | | | | ← | | → | + 196,5% *** |
| Essais n=7 | 1,95±0,34 | Agr 529 2 mg.kg-1 iv | 2 | 3,19±0,38 | Agr 529 2 mg.kg-1 iv + Triton 600 mg.kg-1 iv | 2 | 4,65±0,44 |
| | | | | ← | | → | + 45% ** |

TABLEAU II

Action de l'Agr 529 (2 mg.kg-1), ip) sur l'hypertriglycéridémie provoquee par l'injection intraveineuse de 600 mg.kg-1 de Triton chez le rat (n: nombre d'animaux).

| Animaux<br><br>Dosages | Témoins<br>Soluté NaCl 0,9%<br>+<br>Triton 600 mg.kg-1<br>n=4 | Essais<br>Agr 529 2 mg.kg-1<br>+<br>Triton 600 mg.kg-1<br>n=5 |
|---|---|---|
| Triglycérides<br>mmoles/litre | 6,32±0,92 | 2,90±0,26 |
| | ← - 54% ** → | |

TABLEAU III

ion de l'Agr 529 aux doses de 3 et 30 mg.kg-1 per os et de l'Agr 540 aux doses de 3 et 30 mg.kg-1 per os sur l'hypertriglycéridémie voquée par l'injection intraveineuse de 600 mg.kg-1 de Triton, chez le rat (n: nombre d'animaux).

| Dosage Animaux | Témoins NaCl 0,9% | Témoins Triton 600 mg.kg-1 | Essais Agr 529 3 mg.kg-1 P.O. + Triton 600 mg.kg-1 iv | Essais Agr 529 30 mg.kg P.O. + Triton 600 mg.kg-1 iv | Essais Agr 540 3 mg.kg-1 P.O. + Triton 600 mg.kg-1 iv | Essais Agr 540 30 mg.kg-1 P.O. + Triton 600 mg.kg-1 iv |
|---|---|---|---|---|---|---|
| | n=8 | n=13 | n=4 | n=5 | n=6 | n=8 |
| Triglycérides mmoles/litre | 0,52±0,10 | 7,53±0,49 | 4,87±0,69 | 4,64±0,74 | 5,65±0,46 | 3,67±0,59 |
| | | | - 35% ** | - 38% ** | -25% * | -51% *** |

## Revendications

**1.** Application d'une substance contenant, comme principe actif, une adénosine substituée en $N^6$ de formule I

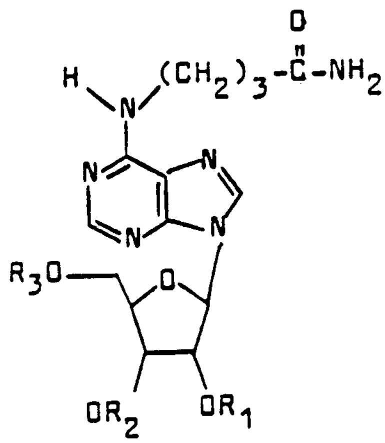

(I)

dans laquelle $R_1$, $R_2$ et $R_3$ représentent chacun l'hydrogène (Agr 529) ou

$$CH_3 - CH_2 - \overset{O}{\overset{\|}{C}} - \qquad (Agr\ 540)$$

pour l'obtention d'un médicament destiné au traitement de l'hyperlipémie.

**2.** Application d'une substance contenant, comme ingrédient actif, une adénosine substituée en $N^6$ de formule I

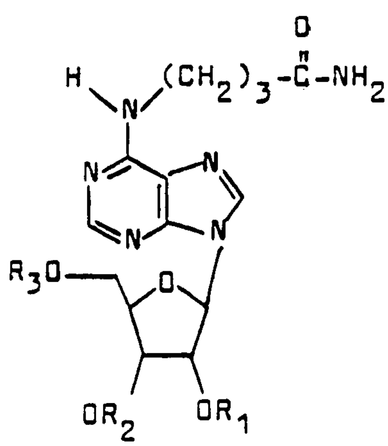

(I)

dans laquelle $R_1$, $R_2$ et $R_3$ représentent chacun l'hydrogène (Agr 529) ou

$$CH_3 - CH_2 - \overset{O}{\overset{\|}{C}} - \qquad (Agr\ 540)$$

pour l'obtention d'un médicament destiné au traitement de l'hypertriglycéridémie.

**3.** Application de la substance selon la revendication 1 ou 2 dans laquelle $R_1$, $R_2$, $R_3$ représentent chacun l'hydrogène.

**4.** Application de la substance selon la revendication 1 ou 2 dans laquelle $R_1$, $R_2$, $R_3$ représentent chacun le reste

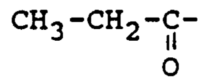

## Claims

1. Application of a substance containing, as the active principle, an adenosine substituted at $N^6$ of formula I

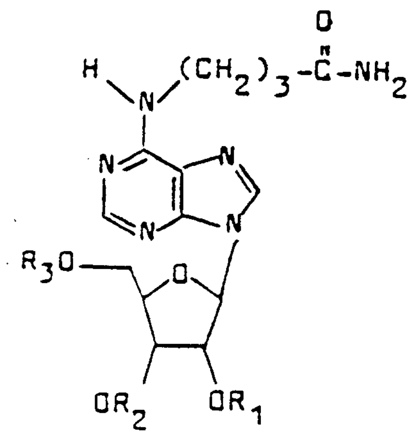

(I)

in which $R_1$, $R_2$ and $R_3$ each represent hydrogen (Agr 529) or

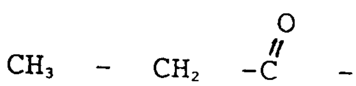

(Agr 540)

for obtaining a medicine intended for the treatment of hyperlipemia.

2. Application of a substance containing, as the active ingredient, an adenosine substituted at $N^6$ of formula I

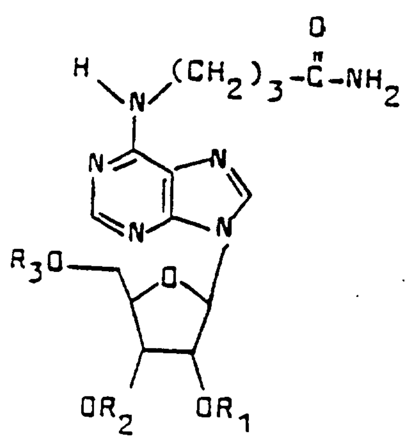

(I)

in which $R_1$, $R_2$ and $R_3$ each represent hydrogen (Agr 529) or

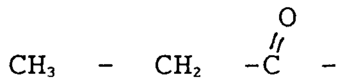 (Agr 540)

for obtaining a medicine intended for the treatment of hypertriglyceridemia.

3. Application of the substance according to Claim 1 or 2 in which $R_1$, $R_2$, $R_3$ each represent hydrogen.

4. Application of the substance according to Claim 1 or 2 in which $R_1$, $R_2$, $R_3$ each represent the remainder

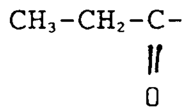

## Patentansprüche

1. Verwendung einer Substanz, die als Wirkstoff ein $N^6$-substituiertes Adenosin der Formel I

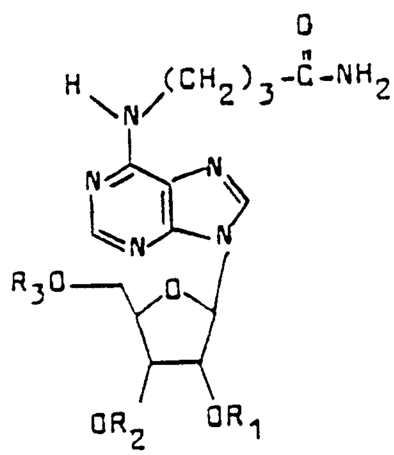

(I)

enthält, in welcher $R_1$, $R_2$ und $R_3$ jeweils für Wasserstoff (Agr 529) oder

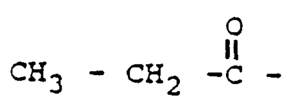 (Agr 540)

stehen, zur Gewinnung eines zur Behandlung von Hyperlipämie bestimmten Arzneimittels.

**2.** Verwendung einer Substanz, die als Wirkstoff ein $N^6$-substituiertes Adenosin der Formel I

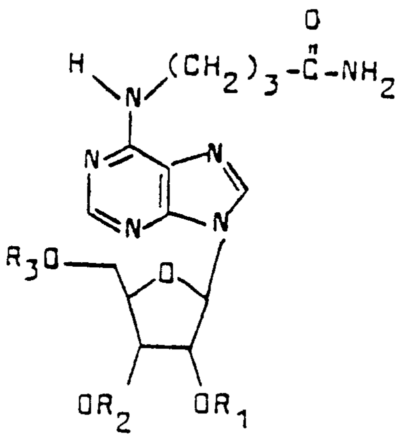

(I)

enthält, in welcher $R_1$, $R_2$ und $R_3$ jeweils für Wasserstoff (Agr 529) oder

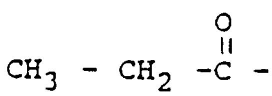

(Agr 540)

stehen, zur Gewinnung eines zur Behandlung von Hypertriglyceridämie bestimmten Arzneimittels.

**3.** Verwendung der Substanz nach Anspruch 1 oder 2, in welcher $R_1$, $R_2$, $R_3$ jeweils für Wasserstoff stehen.

**4.** Verwendung der Substanz nach Anspruch 1 oder 2, in welcher $R_1$, $R_2$, $R_3$ jeweils für den Rest

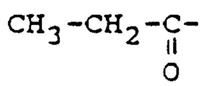

stehen.

FIGURE 1

EFFET DOSE

TRIGLYCERIDES

EFFet rapporté à la dose AGR 529

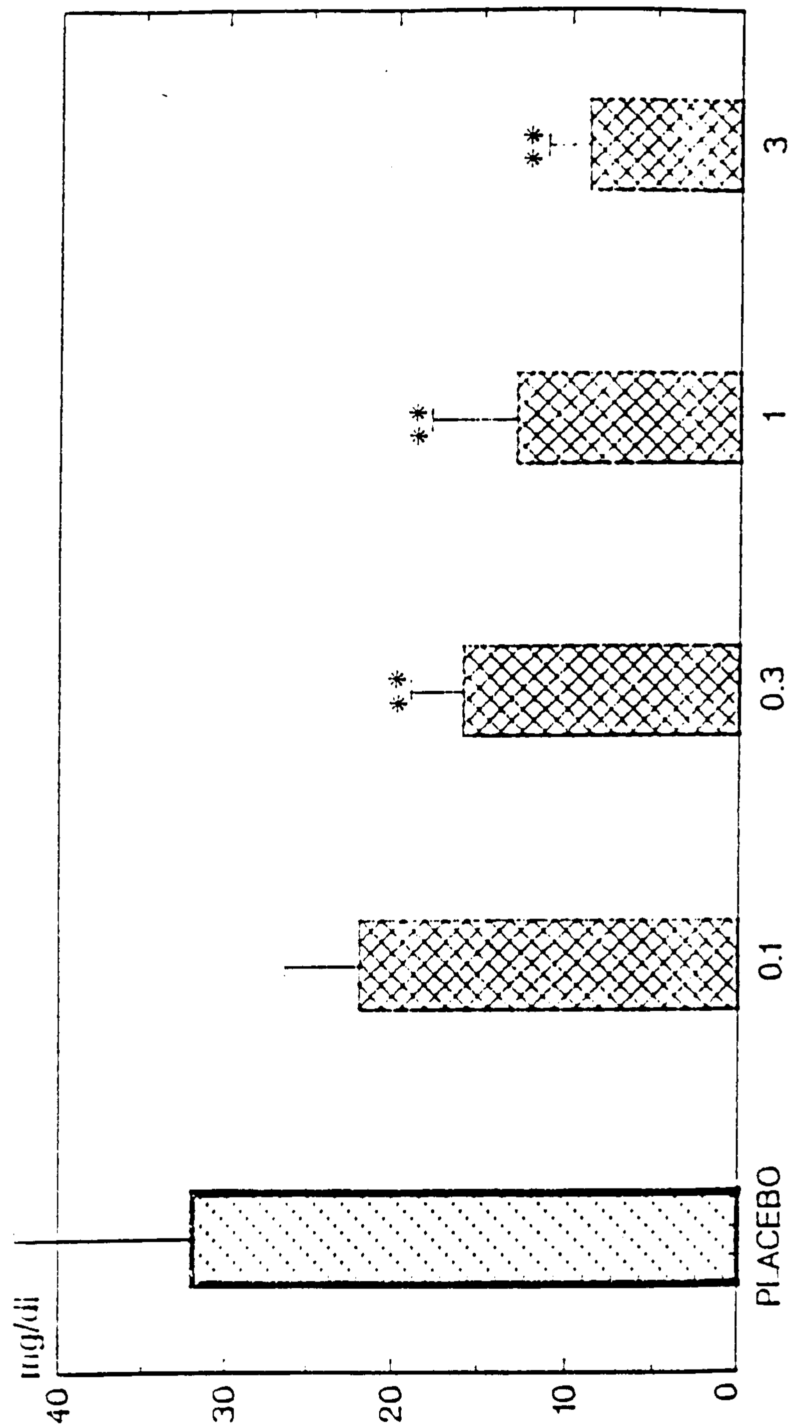


DOSES ( mg/kg i.p.)

* p= ≤ 0.05 ; ** p= ≤ 0.01 par le test de DUNNETT

FIGURE 2

CHOLESTEROL TOTAL

Effet rapporté à la dose AGR 529

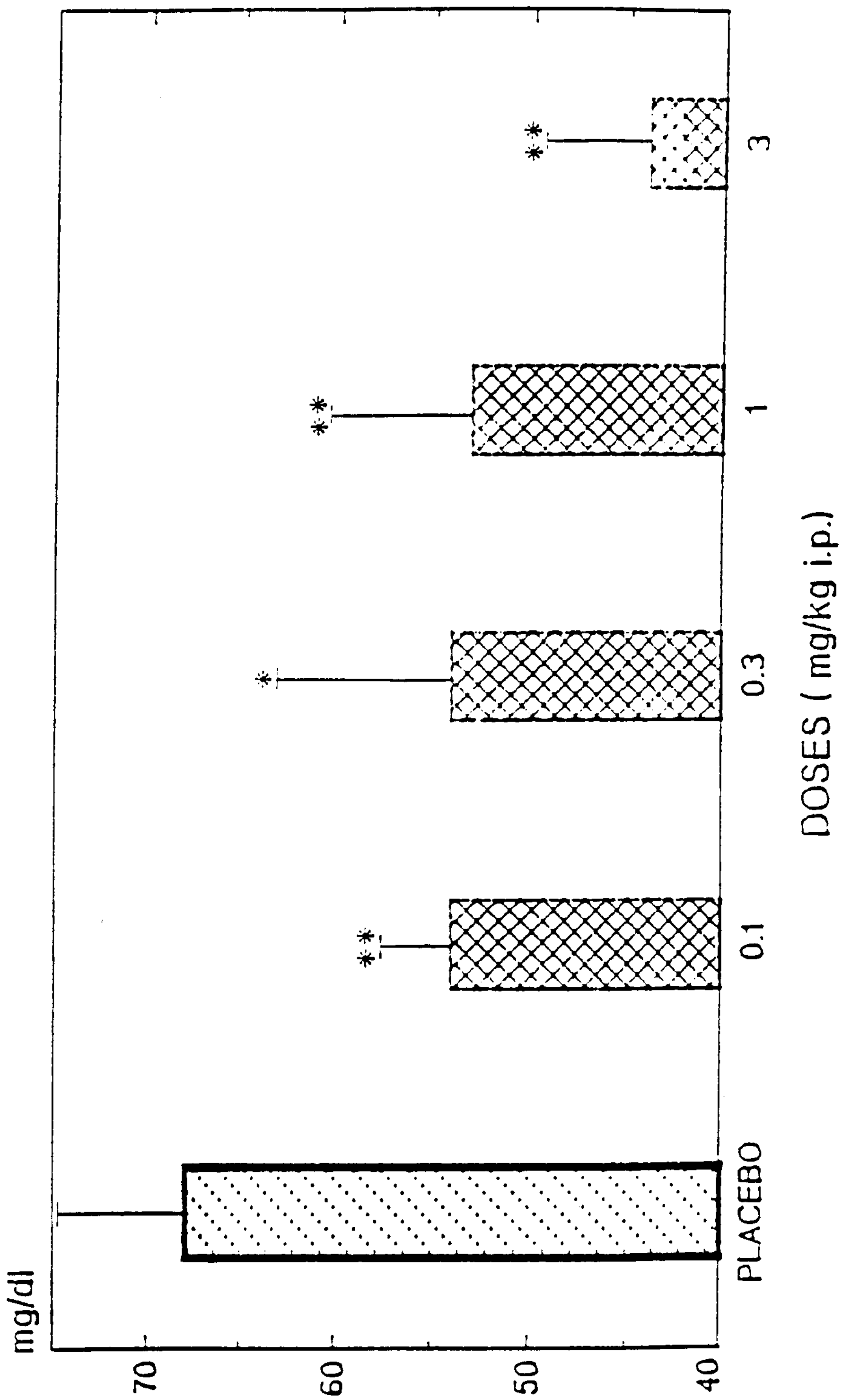


* p= ≤ 0.05 ; ** p= ≤ 0.01 par le test de DUNNETT

FIGURE 3

FFA

Effet rapporté à la dose AGR 529

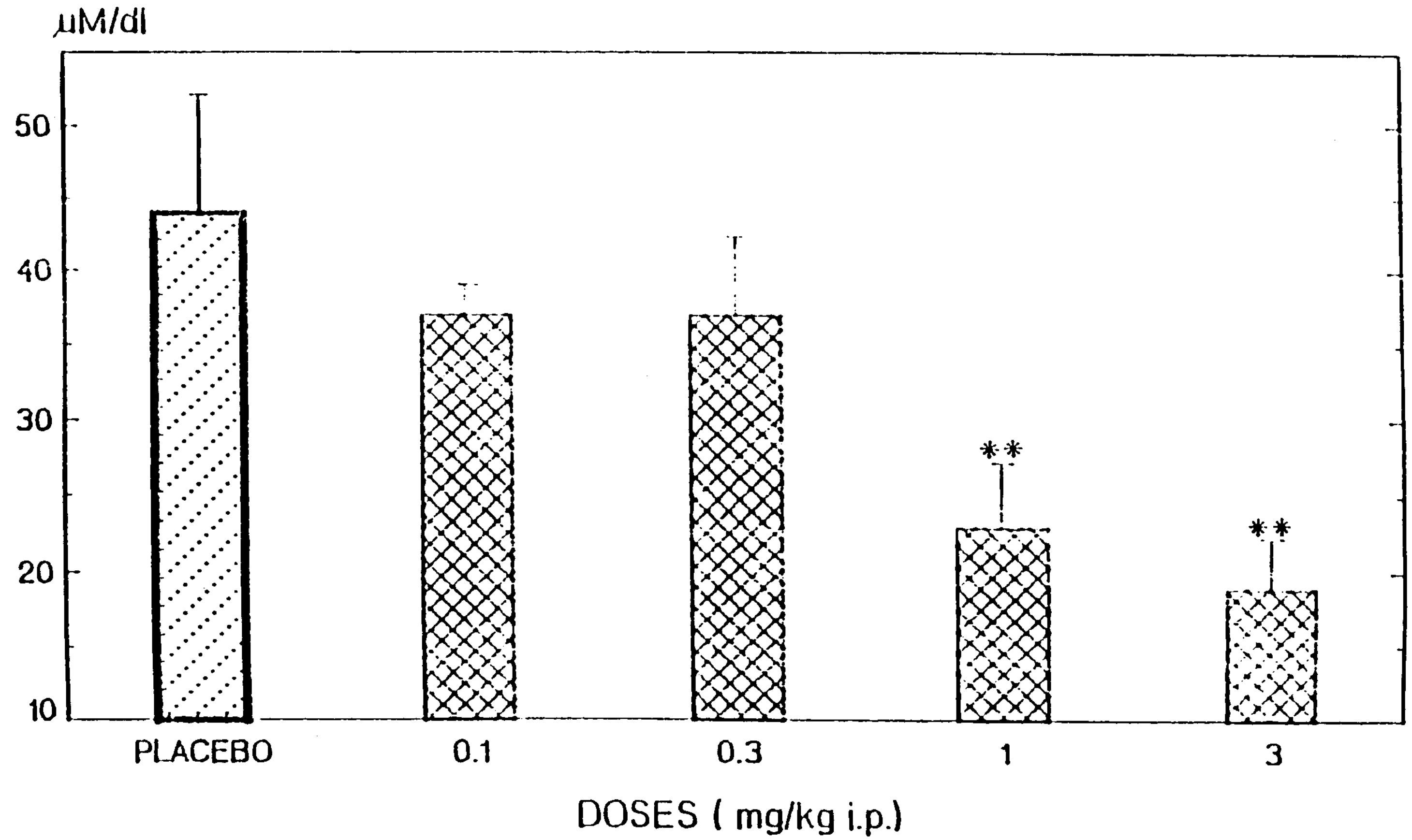


* p=≤ 0.05 ; ** p=≤ 0.01 par le test de DUNNETT

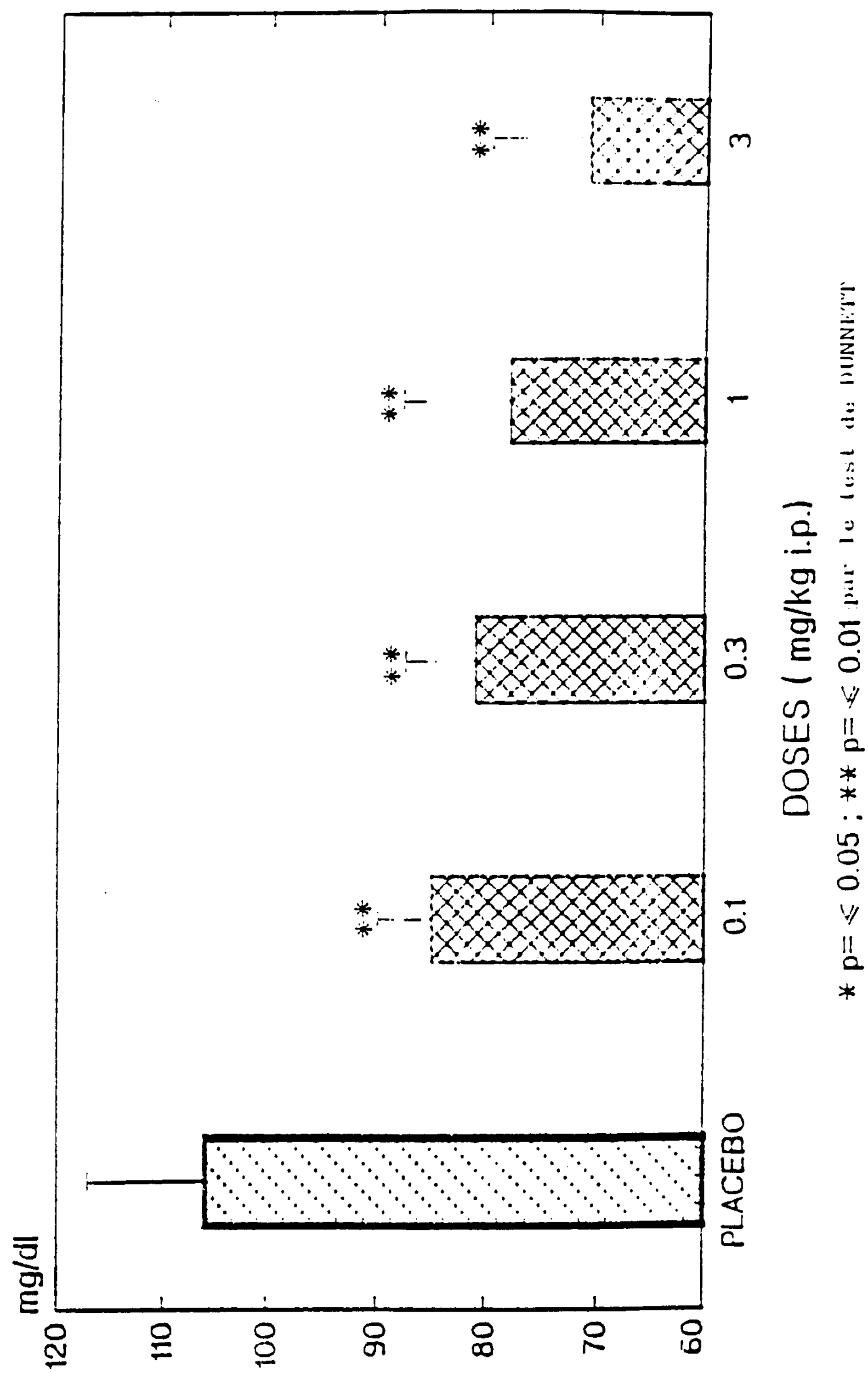


FIGURE 4

PHOSPHOLIPIDES

Effet rapporté à la dose AGR 529

FIGURE 5

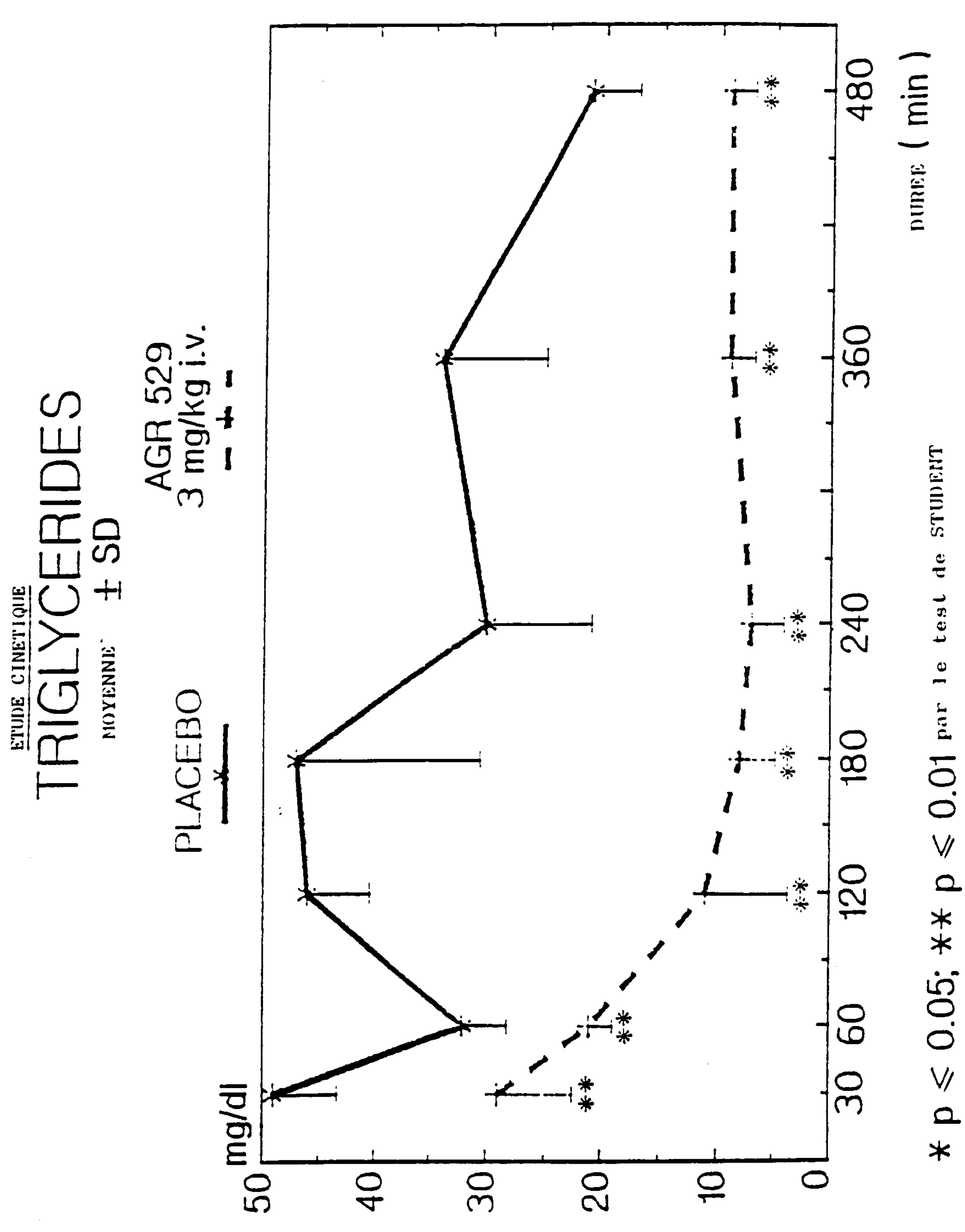
ETUDE CINETIQUE
TRIGLYCERIDES
MOYENNE ± SD
PLACEBO
AGR 529
3 mg/kg i.v.
mg/dl
50
40
30
20
10
0
30
60
120
180
240
360
480
DUREE ( min )
* p ≤ 0.05; ** p ≤ 0.01 par le test de STUDENT

FIGURE 6

CHOLESTEROL TOTAL

MOYENNE ± SD

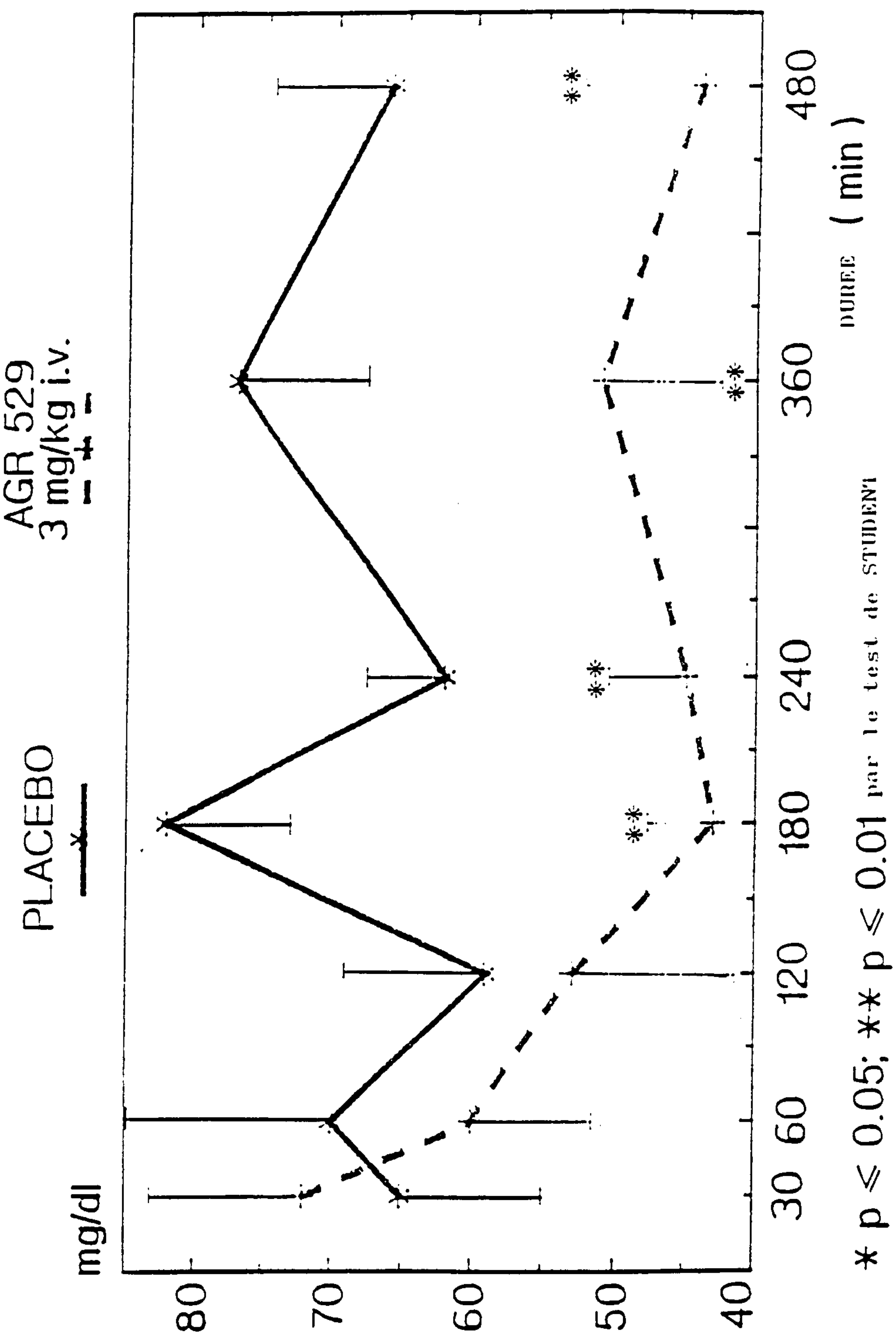


* $p \leq 0.05$; ** $p \leq 0.01$ par le test de STUDENT

FIGURE 7

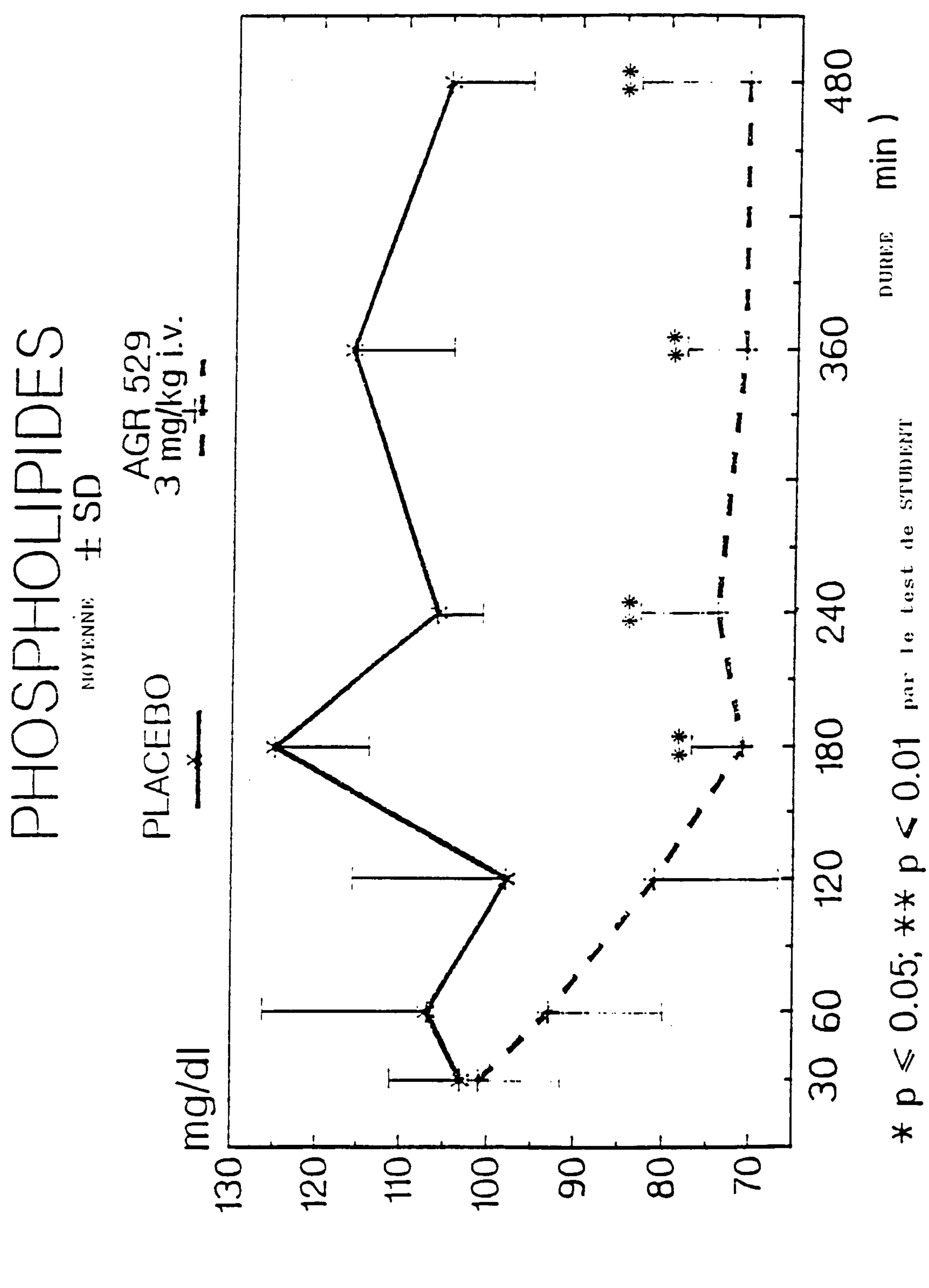
PHOSPHOLIPIDES
MOYENNE ± SD
PLACEBO
AGR 529
3 mg/kg i.v.
mg/dl
130
120
110
100
90
80
70
30 60
120
180
240
360
480
DUREE min )
** ** ** **
* p ≤ 0.05; ** p ≤ 0.01 par le test de STUDENT

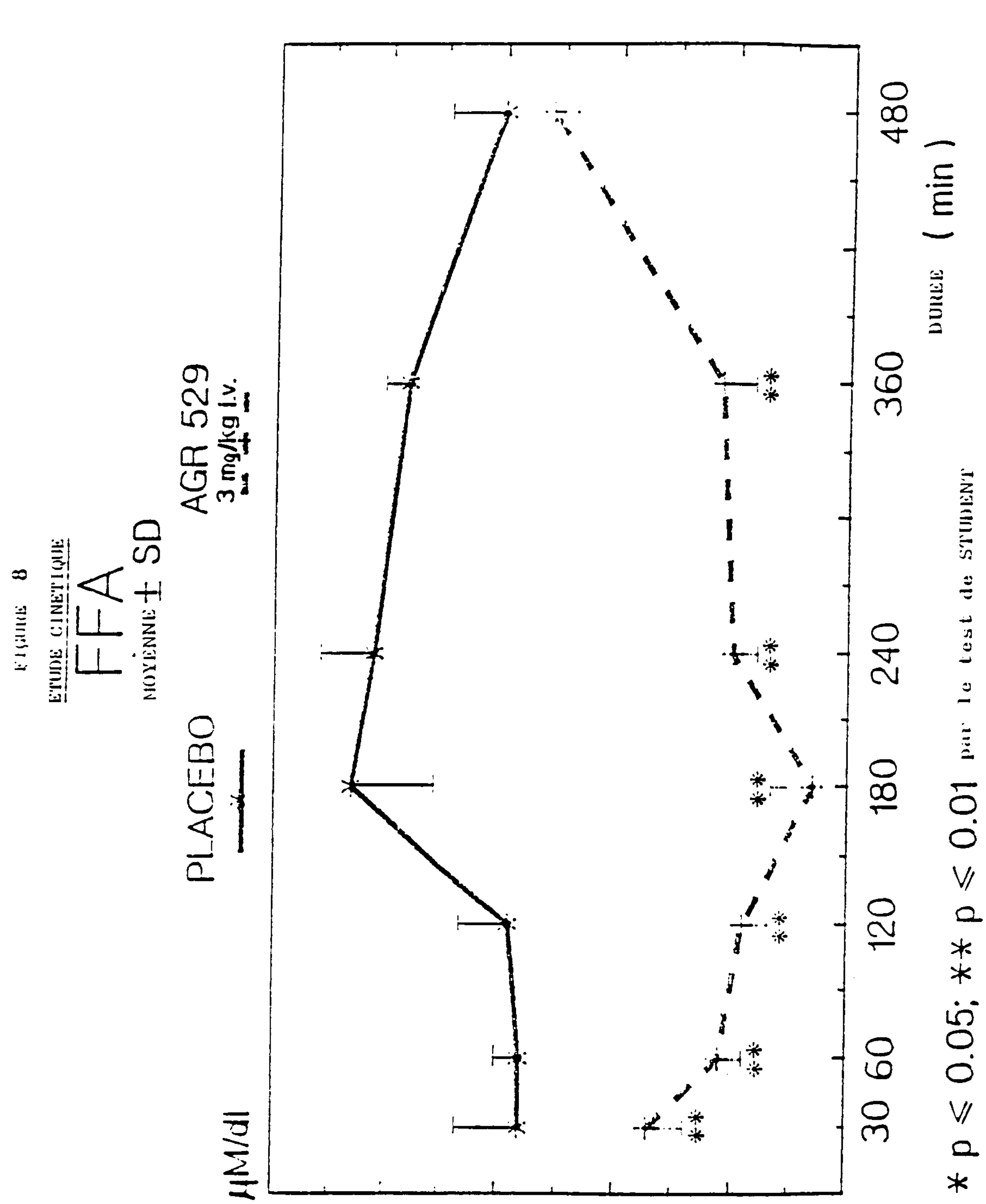
FIGURE 8
ETUDE CINETIQUE
FFA
MOYENNE ± SD
PLACEBO
AGR 529
3 mg/kg i.v.
μM/dl
60
50
40
30
20
10
30
60
120
180
240
360
480
DUREE (min)
* p ≤ 0.05; ** p ≤ 0.01 par le test de STUDENT